# EUROPEAN PATENT SPECIFICATION

(11) **EP 1 961 815 B1**
(45) Date of publication and mention of the grant of the patent: **29.08.2012**
(21) Application number: 06822956.6
(22) Date of filing: 27.10.2006
(51) Int. Cl.: C12N 15/82, C02F 3/32

(54) **Water purification method by plant capabale of accumulating inorganic phosphate**
Verfahren zur Wasseraufreinigung durch Verwendung einer Pflanze, welche Phosphat auf hohem Niveau anreichert
Procédé de purification de l'eau à l'aide d'une plante capable d'accumuler du phosphate inorganique à un taux élevé

(30) Priority: 27.10.2005 JP 2005313225
(43) Date of publication of application: 27.08.2008
(73) Proprietor: Suntory Holdings Limited, Kita-ku, Osaka-shi, Osaka 530-8203 (JP)
(72) Inventor: MATSUI, Keisuke, c/o SUNTORY LIMITED, Mishima-gun, Osaka 6188503 (JP); TOGAMI, Junichi, c/o SUNTORY LIMITED, Mishima-gun, Osaka 6188503 (JP)
(74) Representative: Vossius & Partner
(86) International application number: PCT/JP2006/322038
(87) International publication number: WO 2007/049816

(56) References cited:
- WO-A-00/61822
- WO-A-01/55299
- WO-A-02/15675
- JP-A- 09 056 278
- RUBIO V ET AL: "A conserved MYB transcription factor involved in phosphate starvation signaling both in vascular plants and in unicellular algae" GENES AND DEVELOPMENT, COLD SPRING HARBOR LABORATORY PRESS, PLAINVIEW, NY, US, vol. 15, 1 January 2001 (2001-01-01), pages 2122-2133, XP003012437 ISSN: 0890-9369
- YOSHIWARA R.: 'Shokubutsu ni yoru Kankyo Shufuku-Phytoremediation ni Tsuite' NOGYO DENKA vol. 56, no. 1, 15 January 2003, pages 8 - 12, XP003012436
- RUBIO V. ET AL.: 'A conserved MYB transcription factor involved in phosphate starvation signaling both in vascular plants and in unicellular algae' GENES DEV. vol. 15, 2001, pages 2122 - 2133, XP003012437
- MIURA K. ET AL.: 'The Arabidopsis SUMO E3 ligase SIZ1 controls phosphate deficiency responses' PROC. NATL. ACAD. SCI. USA vol. 102, no. 21, 24 May 2005, pages 7760 - 7765, XP003012438

## Description

### TECHNICAL FIELD

The present disclosure relates to a plant body which highly accumulates inorganic phosphate (for example, petunia, torenia, etc.) and use thereof. In particular, the present disclosure relates to a method for producing a plant body which highly accumulates phosphate, wherein a gene encoding a transcription factor of a gene involved in phosphate starvation reaction is introduced in the plant body, a plant body obtained using the production method, and use of the plant body for water purification.

### BACKGROUND ART

Recently, together with raising of environmental awareness, water pollution has become a big problem. Need for water purification in hydrosphere such as rivers, lakes and ponds has been increased. Water pollution is caused mainly by contamination of toxic substances such as dioxin and heavy metals, excessive inflow of phosphorus, nitrogen, etc., and the like.

Excessive amounts of phosphorus and nitrogen are provided by drainage from agricultural land, livestock excreta, living drainage, industrial effluent and the like. This accelerates eutrophication of hydrosphere, leading to the growth of primary producers such as algae and microorganisms which are direct causes of generation of blue-green algae, red tide organisms, etc. Usually, an element which can be a restriction factor for primary producers is nitrogen or phosphorus. However, in water, nitrogen-fixing algae are ordinarily present, and due to effluent water from land, there are often a lot of nitric acid ions in water. Therefore, with respect to the growth of primary producers, a generally-restrictive element is phosphorus. Phosphorus is not supplied from air. Further, since multivalent and negatively-charged phosphoric ions strongly bind to mineral particles in soil, phosphorus is seldom contained in effluent water from land which is not fertilized (see T. G. Spiro et al., "Chikyu-kankyo no Kagaku (Chemistry of Global Environment)", Japan Scientific Societies Press, 2000). Conversely, the growth of primary producers can be effectively suppressed by removing phosphorus from water.

Eutrophication of lakes, ponds, rivers and oceans due to daily manufacturing performed by human has been questioned for quite some time. In the present circumstances, almost no inorganic ion (e.g., phosphorus, nitrogen or the like) is removed by sewage treatment using the activated sludge method, which is ordinarily employed at sewage-treatment plants, and therefore eutrophication cannot be reduced.

Removing phosphorus and nitrogen from water area is an effective means to solve the problem of water pollution. For the purpose of removal of phosphorus and nitrogen, water purification is carried out using various methods such as physical methods, chemical methods and biological methods. A low-cost and highly-efficient method is desired (Keinosuke Motohashi, "Suishitsu-Joka Manyuaru (Manual for Water purification)", Kaibundo Publishing Co., Ltd., 2001).

Physical and chemical methods (e.g, electrolytic method, crystallization method, aggregation/separation method, etc.) are superior in terms of removal efficiency, but require complicated equipments, continuous use of chemicals and the like, resulting in high cost. Regarding biological methods, the activated sludge method, the Phostrip method (a technique in which the activated sludge method is combined with addition of aggregating agent) and the like are widely used. However, recently, with improvement of purification ability, the problem of increasing costs has been arisen.

An environmental purification method using plants called phytoremediation has been generally utilized. Utilization of plants has been actively attempted in the field of water purification. Since phosphorus and nitrogen, which are causes of water pollution, are nutritive substances essential for plants. Therefore, plants actively absorb these substances from their roots. There are many reports in which phytoremediation was attempted using water plants such as water hyacinth and phragmites, whose ability to absorb phosphorus and nitrogen is relatively high, and which grow well (Keinosuke Motohashi, "Suishitsu-Joka Manyuaru (Manual for Water purification)", Kaibundo Publishing Co., Ltd., 2001). However, high cost for collection of water plants such as water hyacinth, difficulty in management, influence on ecosystem and the like are acknowledged as problems (Keinosuke Motohashi, "Suishitsu-Joka Manyuaru (Manual for Water purification)", Kaibundo Publishing Co., Ltd., 2001). Another problem is that, since most of collected plant biomass cannot be effectively utilized and therefore need to be discarded, extra cost is required. If collected biomass can be directly used as fertilizer, it would be a low-cost processing method. However, absorbing ability of existing plants used in purification has limitation, and therefore, content of phosphorus and nitrogen therein is low. For this reason, such plants cannot be used as fertilizer.

A hydroponic cultivation apparatus using terrestrial flowers and ornamental plants has been developed in order to combine aesthetic purpose with purification (for example, see Japanese Patent Application Laid-open No. Hei 9-56278). However, at present, there is no effective method for treating plant biomass after flowering.

There is a known example, in which vegetables were used for water purification and effective utilization of collected plants was attempted. However, it is unlikely that the consuming public will willingly accept vegetables grown in water-polluted area.

Studies have been conducted to clarify how plants absorb, accumulate and utilize phosphorus. Phosphorus is a nutritive substance essential for plants. Phosphate is initially introduced into a plant from its roots, transported to a vessel, and then provided to the aerial part for utilization. Phosphate is involved in many life phenomena, and is utilized by being changed to the form of phospholipid, nucleotide, phosphorylated protein or the like. In cells, phosphate is accumulated in vacuoles, and is supplied to cytoplasm according to need. In seeds, phosphate is accumulated in the form of more stable inositol hexaphosphate (phytic acid).

Phosphorus can be taken in by plants only in the form of inorganic phosphate (hereinafter referred to as "phosphate"). Other forms of phosphorus such as insoluble organic phosphate cannot be taken in by plants. In general, concentration of phosphate in soil is low, and it is deficient for plants. In such circumstances, plants have developed systems for aggressively taking in phosphate. A key protein for phosphate to be taken in by plants from roots thereof is phosphate transporter, which exists on the cell membrane. Regarding Arabidopsis, 9 high-affinity transporters and 1 chloroplast-located transporter are known. It is reported that uptake of phosphate was accelerated by overexpressing PHT1, which is a cell membrane-located phosphate transporter having high affinity, in a cultured cell of nicotiana, leading to increase in growth rate of the cell (Proc. Natl. Acad. Sci. USA 94,7098-7102,1997). However, it is also reported that, when overexpressing a phosphate transporter in a plant body of Hordeum vulgare, increase in uptake of phosphate was not observed (Func. Plant Biol. 31,141-148,2004). It is considered that just the increase in the number of phosphate transporter does not lead to the increase in the amount of phosphate uptake. Among the above 9 high-affinity phosphate transporters, 2 types strongly express in roots, and it was confirmed that other transporters express in floral organs and the like. Therefore, it is considered that phosphate transporters function for transport within a plant (Plant Physiol. 130,221-233,2002). In a plant, phosphate is transferred from an old leaf to a new leaf for the purpose of effective utilization. It is considered that transporters are also involved in such a phenomenon.

Other than transporters, several phosphate-related genes have been identified by analyzing mutants using Arabidopsis (Trends Plant Sci. 9,548-555, 2004). Most of phosphates taken in from roots are transported to the aerial part via vessels for utilization. However, in the case of PHO1 mutant, transport to the aerial part is inhibited. This indicates that PHO1 protein functions to transport absorbed phosphates from cells of roots to vessels. In the case of pho2 mutant, phosphate is excessively accumulated in the aerial part. It is considered that PHO2 gene encodes protein which controls transport from roots to the aerial part. Thus, though some genes associated with transport of phosphate in plants have been clarified, there are still a lot of unclear points in details of molecular level of phosphate uptake of plants.

Against phosphate deprivation, plants try to take in phosphate as much as possible from the external world by employing various strategies (Annals Botany, 94 323-332, 2004). When sensing the state of phosphate deprivation, plants release organic acid (e.g., citric acid) and acid phosphatase from roots to soil, convert insoluble organic phosphate into soluble inorganic phosphate and try to absorb it. In the state of phosphate deprivation, the form of roots is changed, that is, the number of lateral roots is increased, root hairs are extended, and as a result, a wider range of phosphate can be absorbed. Further, it is known that phosphate deprivation affects the metabolic system in plants, and that as a result, there are cases where gene expression of photosynthetic system is suppressed and accumulation of anthocyanin is increased. Nuclease and phosphatase activity in cells are also increased, and phosphorus existing in cells is reutilized.

Such a series of reactions of plants against phosphate deprivation occur when plants sense the state of phosphate deprivation. Clarification of control factor and control mechanism of these reactions has been waited, but findings thereof are still limited yet. Though many phosphate-related genes have been identified (Trends Plant Sci. 9,548-555, 2004), there is no report known, describing that the ability to absorb phosphate was increased by introducing the genes into plants.

### DISCLOSURE OF THE INVENTION

Under the above-described circumstances, a low-cost and highly-efficient water purification method is desired. In particular, a method for increasing phosphorus accumulation ability of plants to be used for purification and a plant whose phosphorus accumulation ability is increased are desired.

The present inventors made keen examination in view of the above-described problems. Arabidopsis-derived Myb-like transcription factor PHR1 gene was connected downstream of constitutive expression promoter, a terminator was further added thereto, and they were placed on the same vector to prepare a recombinant expression vector. By introducing the prepared vector into petunia and torenia, plants which highly accumulate phosphate were successfully prepared. Thus, the present invention was achieved. The present invention provides a water purification method as defined in the claims.

Further disclosed is :
(1) A plant body which is transformed to express a gene encoding a transcription factor of a gene involved in phosphate starvation reaction.
(2) The plant body according to (1) above, wherein the transcription factor of the gene involved in phosphate starvation reaction is Arabidopsis-derived Myb-like transcription factor PHR1.
(3) The plant body according to (2) above, wherein the Myb-like transcription factor PHR1 is:
   (a) a protein consisting of the amino acid sequence set forth in SEQ ID NO: 2; or
   (b) a protein, which consists of an amino acid sequence in which one or more amino acids are substituted, deleted, inserted and/or added in the amino acid sequence set forth in SEQ ID NO: 2, and which has the function to activate a phosphate starvation response gene.
(4) The plant body according to (1) above, wherein the gene encoding the transcription factor of the gene involved in phosphate starvation reaction is:
   (c) a gene comprising an open reading frame of the nucleotide sequence set forth in SEQ ID NO: 1; or
   (d) a gene, which hybridizes to a polynucleotide consisting of a nucleotide sequence complementary to the nucleotide sequence set forth in SEQ ID NO: 1 or the nucleotide sequence of the open reading frame thereof under stringent conditions, and which encodes a protein having the function to activate a phosphate starvation response gene.
(5) The plant body according to any one of (1) to (4) above, wherein the plant body is petunia, torenia, verbena, nicotiana, rose, impatiens, begonia or cupflower.
(6) A method for producing a plant body comprising transforming the plant body to express a gene encoding a transcription factor of a gene involved in phosphate starvation reaction.
(7) The method according to (6) above, wherein the plant body is transformed by introducing a recombinant expression vector comprising the gene encoding the transcription factor of the gene involved in phosphate starvation reaction into the plant body.
(8) The method according to (6) or (7) above, wherein the transcription factor involved in phosphorus accumulation is Arabidopsis-derived Myb-like transcription factor PHR1.
(9) The method according to (8) above, wherein the Myb-like transcription factor PHR1 is:
   (c) a protein consisting of the amino acid sequence set forth in SEQ ID NO: 2; or
   (d) a protein, which consists of an amino acid sequence in which one or more amino acids are substituted, deleted, inserted and/or added in the amino acid sequence set forth in SEQ ID NO: 2, and which has the function to activate a phosphate starvation response gene.
(10) The method according to (6) or (7) above, wherein the gene encoding the transcription factor of the gene involved in phosphate starvation reaction is:
   (a) a gene comprising an open reading frame of the nucleotide sequence set forth in SEQ ID NO: 1; or
   (b) a gene, which hybridizes to a polynucleotide consisting of a nucleotide sequence complementary to the nucleotide sequence set forth in SEQ ID NO: 1 or the nucleotide sequence of the open reading frame thereof under stringent conditions, and which encodes a protein having the function to activate a phosphate starvation response gene.
(11) The method according to any one of (6) to (10) above wherein the plant body is petunia.
(12) The method according to any one of (6) to (10) above, wherein the plant body is torenia.
(13) A plant body produced using the method according to any one of (6) to (12) above.
(14) A recombinant expression vector comprising a gene encoding a transcription factor of a gene involved in phosphate starvation reaction, which is constituted so that the gene encoding the transcription factor can be expressed by the vector when introduced into a plant.
(15) The vector according to (14) above, wherein the transcription factor of the gene involved in phosphate starvation reaction is Arabidopsis-derived Myb-like transcription factor PHR1.
(16) The vector according to (15) above, wherein the Myb-like transcription factor PHR1 is:
   (a) a protein consisting of the amino acid sequence set forth in SEQ ID NO: 2; or
   (b) a protein, which consists of an amino acid sequence in which one or more amino acids are substituted, deleted, inserted and/or added in the amino acid sequence set forth in SEQ ID NO: 2, and which has the function to activate a phosphate starvation response gene.
(17) The vector according to (14) above, wherein the gene encoding the transcription factor of the gene involved in phosphate starvation reaction is:
   (a) a gene comprising an open reading frame of the nucleotide sequence set forth in SEQ ID NO: 1; or
   (b) a gene, which hybridizes to a polynucleotide consisting of a nucleotide sequence complementary to the nucleotide sequence set forth in SEQ ID NO: 1 or a gene consisting of the open reading frame thereof under stringent conditions, and which encodes a protein having the function to activate a phosphate starvation response gene.
(18) A breeding material for the plant according to any one of (1) to (5) above or (13) above.
(19) A water purification method comprising removing excess phosphorus in hydrosphere using the plant according to any one of (1) to (5) above or (13) above.
(20) A plant cell, which is transformed by introducing the vector according to any one of (14) to (17) above, and by which a plant can be regenerated.

According to the present invention, a low-cost and highly-efficient water purification method as defined by the claims is provided.

According to the method as disclosed herein, the amount of phosphorus accumulation in plants can be increased using genetic recombination technology. According to the present disclosure a plant for water purification, which is advantageous for practical application, and a method for preparing thereof are provided.

Improvement of the water purification ability and the ability to reutilize phosphate can be realized by using the plan as disclosed herein in which the accumulation amount of phosphorus is increased.

Moreover, according to the method as disclosed herein, the phosphate accumulation ability of flowers and ornamental plants for gardening can be increased by means of gene introduction, and thereby water purification using plants having both the beauty and high purification ability can also be realized.

Currently, 80% or more of the amount of imported phosphorus is used as fertilizer. It is most reasonable to reduce phosphorus absorbed by plants to fertilizer. When the phosphorus accumulation amount per plant is increased plants themselves can be utilized as phosphorus fertilizer. Therefore, when using the plant body as disclosed herein with high phosphate accumulation, collected biomass can be turned into fertilizer, and can further be effectively utilized for extraction of phosphorus. By introducing gene into an existing plant having high phosphate accumulation ability using the method for producing a plant which highly accumulates phosphate, a plant which can accumulate more phosphate can also be prepared.

### BRIEF DESCRIPTION OF THE DRAWINGS

Figure 1 is a graph showing the phosphate accumulation amount in a leaf of transformed torenia of the present invention. The longitudinal axis of the graph shows the phosphate amount (mg/gFW).
Figure 2 is a graph showing the phosphate accumulation amount in a leaf of transformed petunia of the present invention. The longitudinal axis of the graph shows the phosphate amount (mg/gFW).
Figure 3 is a graph showing the phosphate accumulation amount in a leaf of hydroponically-cultivated transformed torenia of the present invention. The longitudinal axis of the graph shows the phosphate amount (mg/gFW).
Figure 4 is a graph showing the phosphate accumulation amount per dry weight of the aerial part in hydroponically-cultivated transformed torenia of the present invention. The longitudinal axis of the graph shows the phosphate amount (mg/gDW).
Figure 5 is a graph showing change per day of the phosphate concentration in a hydroponic solution in which transformed torenia of the present invention was cultured. In the graph, the longitudinal axis shows the phosphate concentration (mg/L), and the horizontal axis shows the number of days after exchange of the hydroponic solution.
Figure 6 is a graph showing the phosphate accumulation amount in a leaf of hydroponically-cultivated transformed torenia of the present invention. The longitudinal axis of the graph shows the phosphate amount (mg/gFW).
Figure 7 is a graph showing the fresh weight of the aerial part of hydroponically-cultivated transformed torenia of the present invention. The longitudinal axis of the graph shows the weight (g).
Figure 8 is a graph showing the phosphate accumulation amount in a leaf of transformed nicotiana of the present invention. The longitudinal axis of the graph shows the phosphate amount (mg/gFW).
Figure 9 is a graph showing the phosphate accumulation amount in a leaf of transformed verbena of the present invention. The longitudinal axis of the graph shows the phosphate amount (mg/gFW).

### BEST MODES FOR CARRYING OUT THE INVENTION

Hereinafter, the present invention will be described in detail.

One of known control factors which function when plants get into the state of phosphate deprivation is PHR1 gene of Arabidopsis. PHR1 gene encodes a transcription factor, about which it is considered that it activates transcription of phosphate-related gene in response to the state of phosphate deprivation (Gene Dev. 15, 2122-2133, 2001). In the case of phr1 mutant, phosphate starvation reaction such as anthocyanin accumulation is not shown, and transcription of a plurality of phosphate-related genes is reduced. Therefore, it is considered that PHR1 gene is located upstream of phosphate starvation reaction and increases transcription activity of some genes to increase the ability of plants to take in phosphate. The present inventors thought that, if the state of phosphate starvation of a plant can be maintained by introducing such a control factor of phosphate starvation reaction into the plant, a plant which absorbs phosphate more efficiently may be prepared. The present inventors actually introduced a control factor into a plant, and as a result, high accumulation of phosphate was confirmed, and thus the present invention was completed.

That is, the present disclosure provides a plant body that highly accumulates phosphate, which is transformed so that it expresses a gene encoding a transcription factor of a gene involved in phosphate starvation reaction.

The term "phosphate starvation reaction" as used herein refers to a series of adjustment reactions for obtaining the required amount of phosphate, which are shown by a plant when the plant is kept in the state of phosphate deprivation. Examples of the adjustment reactions include a reaction in which a plant releases organic acid (e.g., citric acid) and acid phosphatase from roots to soil when sensing the state of phosphate deprivation, converts insoluble organic phosphate into soluble inorganic phosphate and tries to absorb it. In the state of phosphate deprivation, the form of roots is changed, that is, the number of lateral roots is increased, root hairs are extended, and as a result, a wider range of phosphate can be absorbed. Further, it is known that phosphate deprivation affects the metabolic system in plants, and that as a result, there are cases where gene expression of photosynthetic system is suppressed and accumulation of anthocyanin is increased. Nuclease and phosphatase activity in cells are also increased, and phosphorus existing in cells is reutilized. The term "phosphate starvation reaction" includes such a series of reactions which occur when a plant senses the state of phosphate starvation.

The term "gene involved in phosphate starvation reaction" as used herein refers to a gene encoding a protein involved in a mechanism in which a plant takes in and accumulates phosphate in the state of phosphate deprivation. For example, a gene encoding a phosphate transporter present on the cell membrane of plant is included in the "gene involved in phosphate starvation reaction". Typical examples thereof include 9 high-affinity transporters (e.g., PHT1) and 1 chloroplast-located transporter of Arabidopsis. However, the "gene involved in phosphate starvation reaction" is not limited thereto. All genes, regarding which increase or decrease in gene expression is confirmed in the state of phosphate deprivation, are included in the "gene involved in phosphate starvation reaction". Note that the phrase "phosphate starvation response gene" as used herein is regarded as synonymous with the phrase "gene involved in phosphate starvation reaction".

The term "transcription factor of a gene involved in phosphate starvation reaction" as used herein refers to a factor which controls transcription of the above-described "gene involved in phosphate starvation reaction" within plant bodies. The "factor which controls transcription" includes factors which suppress or activate transcription, but in the present invention, it mainly refers to factors which activate transcription. However, when a factor which suppresses transcription ultimately acts to promote phosphate starvation reaction by suppressing transcription of a gene involved in phosphate starvation reaction, the factor is included in the "transcription factor of a gene involved in phosphate starvation reaction" used in the present specification. In general, a "transcription factor" functions with the formation of protein complex, but in the present invention, it mainly refers to a protein or peptide which is a component of protein complex. Typical examples of the "transcription factor of a gene involved in phosphate starvation reaction" include, but are not limited to Arabidopsis-derived Myb-like transcription factor PHR1 (SEQ ID NO: 2). A protein, which consists of an amino acid sequence in which one or more (for example, 1 to 20, 1 to 10, or 1 to several (e.g., 6)) amino acids are substituted, deleted, inserted and/or added in the amino acid sequence set forth in SEQ ID NO: 2, and which has the function to activate or suppress a phosphate starvation response gene, is also included in the "transcription factor of a gene involved in phosphate starvation reaction" used in the present specification.

The term "gene encoding a transcription factor of a gene involved in phosphate starvation reaction" as used herein refers to a gene encoding the above-described "transcription factor of a gene involved in phosphate starvation reaction". Typical examples of the "gene encoding a transcription factor of a gene involved in phosphate starvation reaction" include, but are not limited to Arabidopsis-derived Myb-like transcription factor PHR1 gene (Genbank Accession No. AJ310799: SEQ ID NO: 1). A derivative of the above-described PHR1 gene, for example, (a) a gene comprising an open reading frame (or CDS) of the nucleotide sequence set forth in SEQ ID NO: 1; or (b) a gene, which hybridizes to a polynucleotide consisting of a nucleotide sequence complementary to the nucleotide sequence set forth in SEQ ID NO: 1 or the nucleotide sequence of the open reading frame thereof under stringent conditions, and which encodes a protein having the function to activate or suppress a phosphate starvation response gene, is also included in the "gene encoding a transcription factor of a gene involved in phosphate starvation reaction".

Hybridization can be carried out using a publicly-known method or a method according thereto, e.g., a method described in Molecular Cloning Third Edition, J. Sambrook et al., Cold Spring Harbor Lab. Press. 2001. When using a commercially-available library, hybridization can be carried out according to the method described in the instruction for use attached thereto. The phrase "under stringent conditions" means, for example, conditions wherein the sodium concentration is about 19 to 40 mM, preferably about 19 to 20 mM, and temperature is about 50 to 70°C, preferably about 60 to 65°C. Specifically, the case wherein the sodium concentration is about 19 mM and temperature is about 65°C is most preferable.

The phrase "transformed to express a gene" as used herein means that transformation is carried out so that a gene is expressed transiently or stably. Preferably, the phrase means that transformation is carried out to secure stable expression.

Transformation of plant is usually carried out through gene engineering by introducing an expression vector which carries a desired gene into a plant. Further provided is a recombinant expression vector comprising a gene encoding a transcription factor of a gene involved in phosphate starvation reaction.

The vector as disclosed herein usually comprises a suitable expression control region, replication origin, etc. such as a promoter, terminator, etc. depending on the type of a host into which the vector is to be introduced. For example, in order to express a gene in a plant cell, a DNA molecule (expression cassette), which has (1) a promoter which can function in a plant cell; (2) a gene; and (3) a terminator which can function in a plant cell in the form with functionability, is prepared, and the DNA molecule is introduced into the plant cell. Such a DNA molecule may contain a DNA sequence for further enhancing transcription, e.g., an enhancer sequence, in addition to a promoter. Examples of promoters used are not particularly limited as long as they can function in plant cells, and include, for example, 35S (Shcell, J. S., 1987, Science, 237: 1176-1183), Nos (Schell, J. S., 1987, Science, 237: 1176-1183), rbcS (Benefy, P. N. and N-H. Chua, 1989, Science, 244: 174-181), PR1a (Ohshima, M. et al., 1990, Plant Cell, 2: 95-106), ADH (Benefy, P. N. and N-H. Chua, 1989, Science, 244: 174-181), patatin (Benefy, P. N. and N-H. Chua, 1989, Science, 244: 174-181), Cab (Benefy, P. N. and N-H.Chua, 1989, Science, 244: 174-181), and PAL (Lian, X. et al., 1989, Proc. Natl. Acad. Sci. USA, 86: 9284-9288), etc.

Construction of the above-described vectors can be carried out according to ordinary methods using publicly-known restriction enzymes and the like. For example, when using Agrobacterium, a binary vector such as pBI121 can be used, and when using a particle gun, an E. coli vector such as pUC19 can be used. Further, for example, by selecting a plant cell transformed with the vector using a marker gene such as an antibiotic-resistant gene and redifferentiating the plant cell using conditions such as a suitable plant hormone, a plant body transformed with a target gene can be obtained. Note that the term "plant" as used herein is regarded as synonymous with the term "plant body".

Introduction of a vector into a plant cell can be carried out using various methods known in the art. For example, indirect introduction methods utilizing Agrobacterium tumefaciens, Agrobacterium rhizogenes, etc. (Heiei, Y et al., Plant J., 6, 271-282, 1994; Takaiwa, F. et al., Plant Sci. 111, 39-49, 1995), and direct introduction methods typified by electroporation methods (Tada, Y et al., Theor. Appl. Genet, 80, 475, 1990), polyethylene glycol methods (Datta, S. K. et al., Plant Mol Biol., 20, 619-629, 1992), particle gun methods (Christou, P. et al., Plant J. 2,275-281,1992; Fromm, M. E., Bio/Technology, 8, 833-839, 1990), etc. can be used. Examples of plant cells to which gene introduction is applied are not particularly limited as long as a plant body can be regenerated therefrom, and include, for example, suspension-cultured cell, callus, protoplast, cell constituting a section of a leaf and the like. Further provided is a plant cell, which is transformed so that it expresses the gene encoding a transcription factor of a gene involved in phosphate starvation reaction, and from which a plant body can be regenerated.

A transformed plant cell can be subjected to regeneration to produce a plant body. A transformed transgenic plant can be prepared by this means. A plant body that highly accumulates phosphate, which is transformed so that it expresses the gene encoding a transcription factor of a gene involved in phosphate starvation reaction, can be prepared in the same way.

Further provided is a method for producing a plant body that highly accumulates phosphate, which comprises transforming a plant body so that it expresses a gene encoding a transcription factor of a gene involved in phosphate starvation reaction. Preferably, the above-described plant body is transformed by introducing a recombinant expression vector, which comprises the above-described gene encoding a transcription factor of a gene involved in phosphate starvation reaction, into the plant body.

Examples of preferable plant species for transformation with the vector includes those whose transformant can be obtained. Specifically, examples of such plant species include petunia, torenia, verbena, nicotiana, rose, impatiens, begonia, cupflower, chrysanthemum, carnation, antirrhinum, cyclamen, orchid, Eustoma russellianum, freesia, gerbera, gladiolus, gypsophila, kalanchoe, lily, pelargonium, geranium, tulip and the like. In particular, petunia, torenia, verbena, nicotiana, rose, impatiens, begonia and cupflower are preferable. Among them, plant species, which have high growth rate and high phosphorus concentration in plant bodies, and which grow by being adapted to a wide range of phosphate concentration in water, are desired

In addition to plant bodies in which phosphate is highly accumulated as a result of introducing the vector of the present invention into the plant bodies and expressing the vector therein, plant bodies obtained from breeding materials thereof (e.g., seed, posterity, cut flower, tuberous root, stem tuber, spike, etc.) are also included.

Futher provided is a water purification method comprising removing excess phosphorus in hydrosphere using the above-described plant.

The phrase "excess phosphorus in hydrosphere" as used herein refers to phosphorus at concentrations higher than the concentration at or below which balance between primary producers and secondary producers can be maintained. Specifically, the phrase refers to phosphorus at concentrations higher than the concentration at or below which generation of blue-green algae and red tide organisms is suppressed. For example, in the case of the environmental standards defined by Organisation for Economic Co-operation and Development (OECD), nutritional states of lakes are classified depending on total phosphorous concentrations. Lakes having total phosphorous concentration of 0.01mg/l or less are classified into oligotrophic lakes, and lakes having total phosphorous concentration of 0.035mg/l or more are classified into eutrophic lakes. Under conditions provided by eutrophic lakes, blue-green algae and the like tend to be generated, although it cannot be categorically described since factors other than phosphorus are also associated with the matter. The term "hydrosphere" as used herein refers to freshwater area. Typical examples thereof include lakes, ponds, rivers, etc.

Preferably, the plant body as disclosed herein is planted and grown in a place which requires water purification with removal of excess phosphorus, such as a reservoir, a storage reservoir for agriculture, a pond in a park, etc., into which polluted water is poured, and absorbs excess phosphorus contained in water existing in the place, thereby removing phosphorus from environments (e.g., hydrosphere, soil, etc.). After that, biomass of the plant that highly accumulates phosphorus is collected, and is preferably used as fertilizer. In order to use as fertilizer, collected biomass is preferably subjected to treatments such as powdering by means of drying/pulverization. If a plant itself shows high accumulation of phosphate, it can be turned in agricultural land or field without processing and can be directly used as fertilizer. Thus, according to the plant body biomass after collected, which had been the major issue of plants for water purification, can be effectively reutilized by converting it to fertilizer or by extracting phosphorus from biomass for reutilization.

Hereinafter, the present invention will be specifically described by way of illustrative examples.

### EXAMPLES

In the following examples, the molecular biological technique is employed according to the method described in WO96/25500 or Molecular Cloning (Sambrook et al. (1989) Cold Spring Harbour Laboratory Press), unless otherwise specified.

### Example 1:_ Construction of PHR1 Expression Vector

PHR1 gene was subcloned into pCR2.1 vector using a TOPO-TA cloning kit (Invitrogen) according to the instruction manual. A product amplified by PCR reaction using primers PHRf (5'-ATGGAGGCTCGTCCAGTTCAT-3 , (SEQ ID NO: 3)) and PHRr (5'-TCAATTATCGATTTTGGGACGC-3' (SEQ ID NO: 4)) was subcloned to obtain pSPB1892. Binary vector pSPB176 having a cauliflower mosaic virus 35S (El₂35S) promoter in which the enhancer sequence is repeated and a nopaline synthase (nos) terminator was digested with BamHI and Sall to obtain pSPB176A. pSPB1892 was digested with BamH1 and Xhol, and the obtained PHR1 gene fragment was inserted into pSPB176A to obtain pSPB1898.

Binary vector pSPB2311 having a promoter in which the enhancer sequence of 35S promoter is connected to manopine synthase promoter (Mac) and a manopine synthase (mas) terminator was digested with Smal to obtain pSPB2311A. pSPB1892 was digested with EcoRI, and the smoothed fragment was inserted into pSPB2311A to obtain pSPB2314.

In order to enhance transcription-activating ability of PHR1, a vector for chimeric protein expression, in which the transcription-activating region of VP16 protein of human herpes simplex virus is connected to PHR1 gene, was also prepared. A product amplified by PCR reaction using primers BamPHRf (5'-AGCTGGATCCATGGAGGCTCGTCCAG-3' (SEQ ID NO: 5)) and SpePHRr (5'-CAGTACTAGTATTATCGATTTTGGGA-3' (SEQ ID NO: 6)) was digested with BamHI and Spel to obtain DNA fragment A. A product amplified by PCR reaction using primers SpeVP16f (5'-AGCTACTAGTGGCCCCCGGACCGATG-3' (SEQ ID NO: 7)) and KpnVP16r (5'-CAGTGGTACCTTACCCACCGTACTCG-3' (SEQ ID NO: 8)) was digested with SpeI and KpnI to obtain DNA fragment B. Three-point ligation was carried out using pSPB2311 which was digested with BamHI and Kpnl, DNA fragment A and DNA fragment B to prepare vector pSPB2377 in order to express a chimeric protein, in which a peptide consisting of 79 amino acid residues derived from VP16 is connected to the C-terminal side of PHR1 protein. PHR1 genes on pSPB1898, pSPB2314 and pSPB2377 are under the control of a constitutive promoter.

### Example 2: Preparation of Transformant

Subsequently, Agrobacterium tumefaciens strain Ag10 was transformed using pSPB1898 or pSPB2314 or pSPB2377 based on a publicly-known method (Plant J. 5, 81, 1994), and petunia (Petunia hybrida) and torenia (Torenia hybrida) were infected with the transformed Agrobacterium having pSPB1898 or pSPB2314 or pSPB2377. RNAs were extracted from leaves of the obtained recombinant plants using RNeasy Plant Mini Kit (Qiagen), and strains in which the introduced gene was expressed were selected by means of RT-PCR according to the ordinary method.

### Example 3: Phosphate Accumulation Amount of Transformant

### (1) Method for Measuring Phosphate Concentration

Phosphate concentration was measured according to the method of Ames (Methods EnzymoL 8, 115-118, 1966), which was partially modified. Leaves were weighed (by about 100 mg) and subjected to sampling. The sample was encapsulated into a 2 ml tube for shaking/crushing together with zirconia beads having the diameter of 4 mm, and frozen at -80°C. The frozen sample was taken out into room temperature, 500 µl of 1% (v/v) acetic acid was put into the tube, and the mixture was shaken and crushed for 6 minutes using a shaking/crushing machine (Qiagen). After crushing, the mixture was centrifuged at 15,000 rpm for 5 minutes using a desktop centrifuge to obtain 500 µl of supernatant as a phosphate extract. This phosphate extract was prepared using distilled water (10 to 100-fold dilution) so that the final amount was adjusted to 800 µl. To this solution, 160 µl of reagent for measuring phosphate (the composition thereof will be described later) was added, and the mixture was stirred well and left for 10 minutes. Abs880 was measured using a spectrophotometer, and the phosphate concentration was determined by comparison with the standard curve drawn using the standard solution. The amount of phosphate contained in 1g of leaf was calculated based on the obtained phosphate concentration and the weight of the sample.

### (Composition of reagent for measuring phosphate)

| | |
|---|---|
| 2.5M sulfuric acid | 10ml |
| 0.1 M ascorbic acid | 6ml |
| 0.27g/100ml antimonyl potassium tartrate | 3ml |
| 8g/100ml ammonium molybdate | 6ml |

The above-described materials are mixed together to provide 20 ml of solution. Ascorbic acid is prepared as needed.

### (2) Amount of phosphate in transformed torenia and petunia

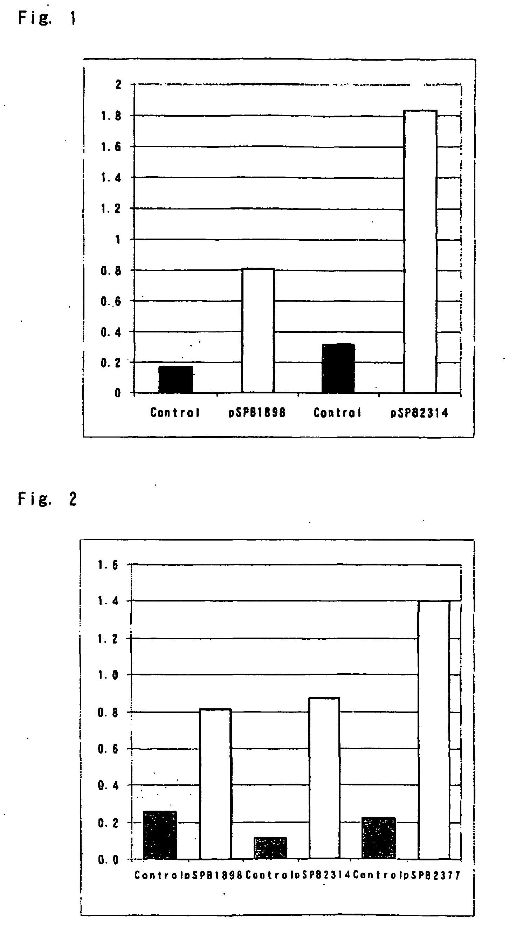

Using leaves of transformant obtained, the phosphate concentration was measured according to the above-described method, and the content of phosphate per fresh weight was determined. As shown in Table 1 and Figure 1, in the case of transformed torenia, both pSPB1898 using 35S promoter and pSPB2314 using Mac promoter showed phosphate accumulation amount about 5 times higher than that of the wild type.

**Table 1**

| | Introduced gene | Amount of phosphate (mg/gFW) | Relative value (/Control) |
|---|---|---|---|
| Torenia | Control | 0.17 | 1 |
| | pSPB1898 | 0.81 | 4.76 |
| | Control | 0.32 | 1 |
| | pSPB2314 | 1.84 | 5.75 |

As in the case of torenia, in the case of transformant of petunia, the PHR1 gene-introduced strain showed higher phosphate accumulation compared to the wild type (Table 2 and Figure 2). The transformant of petunia, into which pSPB2377 that expresses PHR1 connected to the transcription activation site of VP16 was introduced, showed the highest phosphate accumulation. It was considered that the transcription activation site of VP16 acts effectively in petunia.

**Table 2**

| | Introduced gene | Amount of phosphate (mg/gFW) | Relative value (/Control) |
|---|---|---|---|
| Petunia | Control | 0.26 | 1 |
| | PSPB1898 | 0.81 | 3.12 |
| | Control | 0.11 | 1 |
| | pSPB2314 | 0.87 | 7.91 |
| | Control | 0.22 | 1 |
| | pSPB2377 | 1.4 | 6.36 |

### Example 4: Amount of phosphate of transformant in the case of hydroponic culture (1) Amount of phosphate per fresh weight of hydroponically-cultivated torenia

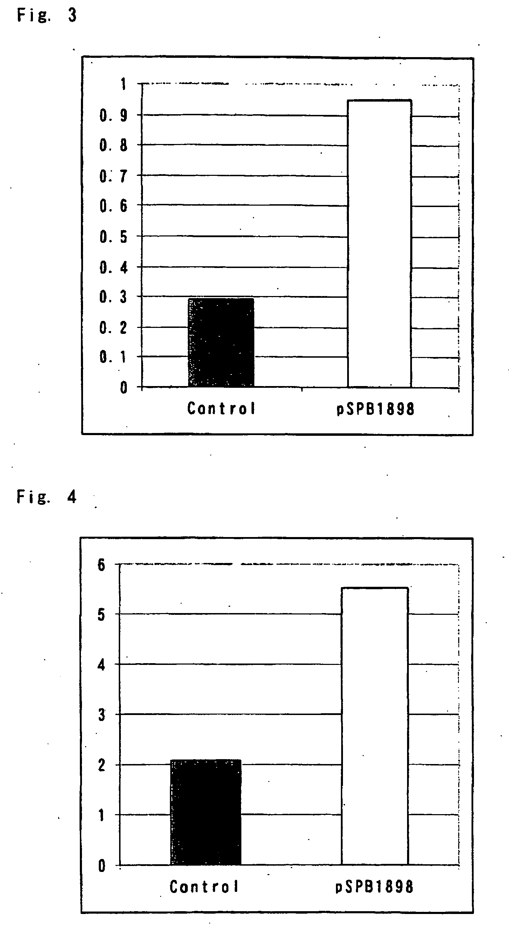

Hydroponic culture was conducted using pSPB1898 transformant of torenia, and it was examined whether or not the ability to absorb phosphate also increases in the case of hydroponic culture. Leaves of torenia, which was hydroponically-cultivated in a hydroponic solution having the phosphate concentration of 5mg/L for about one month, were sampled, and the phosphate concentration was measured. As shown in Table 3 and Figure 3, the transformant showed the phosphate accumulation amount about 3 times higher than that of the wild type. Thus, increase in absorbing ability attributed to PHR1 gene was also confirmed when used in hydroponic culture.

**Table 3**

| | Introduced gene | Amount of phosphate (mg/gFW) | Relative value (/Control) |
|---|---|---|---|
| Hydroponically cultivated torenia | Control | 0.29 | 1 |
| | SPB1898 | 0.95 | 3.28 |

There was no difference of morphology between the hydroponically-cultivated pSPB1898 transformant and the wild type. Moreover, there was no difference of plant weight after culture for a certain period between them. Thus, it was suggested that excessively-absorbed phosphate is not used for plant growth but is accumulated in a plant body as it is. It was also suggested that constitutive expression of PHR1 does not affect plant growth.

### (2) Amount of phosphate per dry weight of hydroponically-cultivated torenia

In order to confirm whether or not the phosphate accumulation amount was increased in entire plant body, the whole aerial part of pSPB1898 of torenia which was hydroponically-cultivated with the phosphate concentration of 5 mg/L for about 2 months was collected and dried at 80°C for about 2 days, and the phosphate accumulation amount per dry weight was measured (Table 4 and Figure 4). 3 individuals of wild type and 3 individuals of transformant were dried, and their phosphate concentration was measured to calculate average values. As a result, in the case of dried torenia, the transformant showed the phosphate accumulation amount about 2.5 times higher than that of the wild type. Thus, it was confirmed that the phosphate accumulation amount increased in entire plant body.

**Table 4**

| | Fresh weight (g) | Dry weight (g) | Amount of phosphate (mg/gDW) | Average |
|---|---|---|---|---|
| Control | 28.78 | 2.67 | 2.08 | 2.07 |
| | 29.85 | 2.70 | 2.14 | |
| | 26.41 | 2.53 | 2.00 | |
| pSPB1898 | 25.63 | 2.20 | 5.77 | 5.52 |
| | 35.40 | 3.16 | 4.77 | |
| | 36.05 | 3.20 | 6.02 | |

Thus, it was clarified that plants' ability to accumulate phosphate can be enhanced by introducing PHR1 gene into plants to allow to be overexpressed.

### Example 5: Amount of phosphate absorbed from hydroponic solution

In order to clarify the difference of the amount of phosphate absorbed from hyroponic solution, the following comparison experiment was conducted. Wild-type torenia or PHR1 -introduced transformed torenia was planted and grown on a support made of foamed polystyrene having holes which was floated on 5 1 of hydroponic solution (deionized water in which various salts were dissolved) in a plastic container (23 x 23 x 11.5 cm). The phosphorus concentration in the hydroponic solution was set to be 5 mg/L. 4 plants were used in each container. The hydroponic solution was sampled per day, and the phosphate concentration was measured according to the above-described method. Since the fluid volume of the hydroponic solution is decreased due to transpiration in leaves and evaporation from water surface, deionized water was replenished every forth day.

### (Preparation of hydroponic solution)

In 10 L (phosphorus concentration: 5mg/L):

| | |
|---|---|
| 1M KNO₃ | 5ml |
| 1M MgSO₄ | 2ml |
| 1M Ca(NO₃)₂ | 2ml |
| 20mM Fe-EDTA | 2.5ml |
| Micronutrient | 1ml |
| 1M KPO₄ buffer (pH 5.5) | 1.61 ml |

Liquid composition of micronutrient (in 50ml)

| | |
|---|---|
| 350mM H₃BO₃ | 10ml |
| 140mM MnCl₂ | 5ml |
| 50mM CuSO₄ | 500µl |
| 100mM ZnSO₄ | 500µl |
| 20M Na₂MoO₄ | 500µl |
| 5M NaCl | 100µl |
| 10mM CoCl₂ | 50µl |

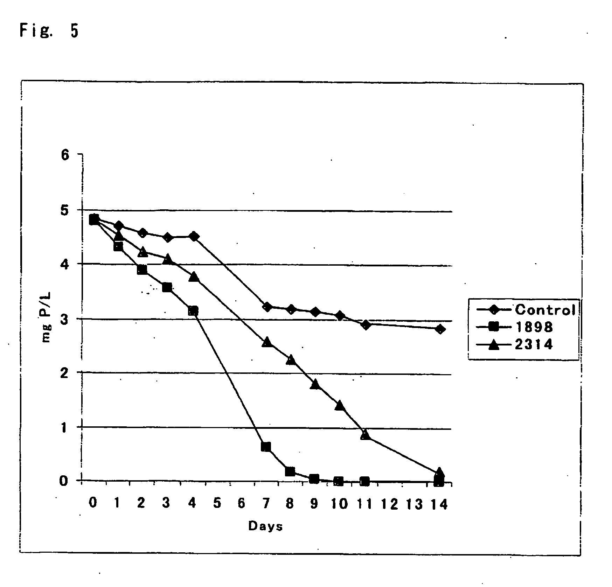

The measurement results showed that the PHR1 transformant has superior ability to absorb phosphorus compared to the wild type (Table 5 and Figure 5). Both the transformants pSPB1898 and pSPB2314 absorbed most of phosphorus in the solution within 14 days after starting culture, and showed high absorption ability. Moreover, since most of phosphate was absorbed, it was considered that phosphate is actively absorbed at low concentration levels.

**Table 5**

| | Number of days | 0 | 1 | 2 | 3 | 4 | 7 | 8 | 9 | 10 | 11 | 14 |
|---|---|---|---|---|---|---|---|---|---|---|---|---|
| Phosphate concentration in hydroponic solution (mg/L) | Control | 4.7 | 4.6 | 4.5 | 4.4 | 4.4 | 3.2 | 3.2 | 3.1 | 3.1 | 2.9 | 2.8 |
| | pSPB1898 | 4.7 | 4.2 | 3.8 | 3.5 | 3.1 | 0.6 | 0.2 | 0 | 0 | 0 | 0 |
| | pSPB2314 | 4.7 | 4.4 | 4.1 | 4 | 3.7 | 2.6 | 2.3 | 1.8 | 1.4 | 0.9 | 0.2 |

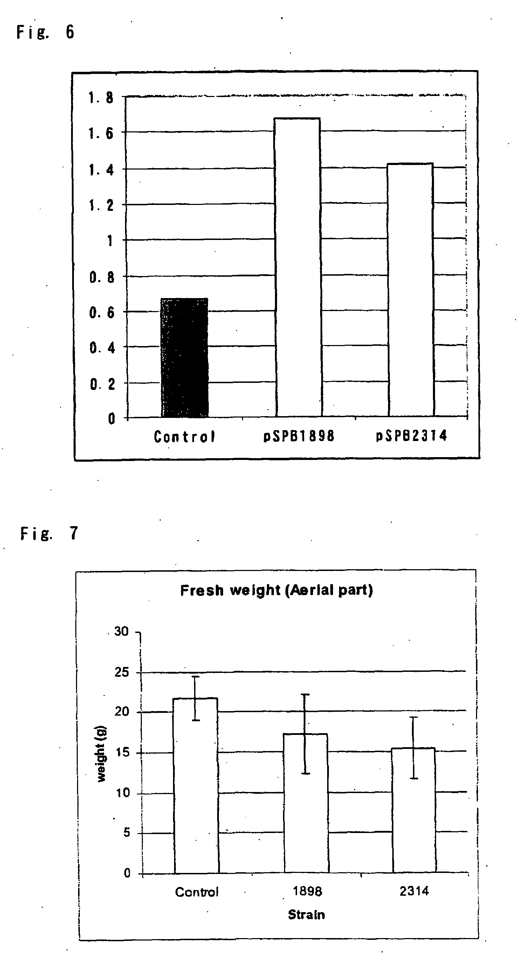

The amount of phosphate in leaves of plants cultured for 4 weeks (2-week test was doubled) was analyzed. The results showed the same tendency as those of the torenia and petunia in Examples 3 and 4, and the transformants highly accumulated phosphate (Table 6 and Figure 6). Figure 7 shows results of comparison in which the fresh weight of the aerial part of every strain of torenia after 4-week hydroponic culture was measured, and their average values were compared. It is understood that the weights of the transformants are a little smaller than that of the wild type, but there is no significant difference. In view of this, it was considered that transformants do not utilize absorbed phosphate for their growth, but accumulate and hold it.

**Table 6**

| Introduced gene | Amount of phosphate (mg/gFW) | Relative value (/Control) |
|---|---|---|
| Control | 0.67 | 1 |
| pSPB1898 | 1.67 | 2.49 |
| pSPB2314 | 1.42 | 2.12 |

The above-described results show that transformed plants into which PHR1 gene is introduced have enhanced ability to absorb phosphate and have obtained characteristics suitable for water purification using plants.

### Example 6: PHR1 gene introduction into other plants

In order to confirm whether or not increase in phosphate absorption ability attributed to PHR1 gene is observed in other plants, PHR1 gene was introduced into impatiens, begonia, nicotiana, verbena, cupflower and rose.

### (PHR1 gene introduction into impatiens and begonia)

Transformation of impatiens (Impatiens walleriana) was conducted using breed types Glitter Red (Sakata Seed Corporation) and Tempo Pink (Takii & Co., Ltd.) basically according to US Patent 6,121,511. Shoot apex, node, petiole and leaf disc cut from in vitro seedling of impatiens were precultured in a preculture liquid medium (1mg/L of TDZ-added MS medium) for 5 days, and then left to stand on a preculture solid medium (MS medium to which 0.05mg/L of NAA, 6mg/L of Zeatin and 0.3% gellangum were added) for 48 hours. After that, the samples were infected with Agrobacterium (Agrobacterium tumefaciens Ag10 strain) into which pSPB2314 was introduced, and cultured on a selective medium (MS medium to which 0.05mg/L of NAA, 6mg/L of Zeatin, 100mg/L of Kanamycin, 500mg/L of Carbenicillin, 100mg/L of Cefotaxime, and 0.3% gellangum were added) for 4 to 8 weeks to obtain shoots (Table 7). In the case of the sample of leaf disc, the sample easily browned and no shoot was obtained. In the case of the samples of shoot apex and node, many shoots were obtained from one explant, but it was considered that many false-positive ones were included therein. Appearance of shoots from petiole requires considerable time, and they are small in number. However, it appears to be most certain.

**Table 7**

| Number of shoots of impatiens | | | |
|---|---|---|---|
| Breed type | Origin of explants | Number of infected explants | Number of shoots obtained |
| Glitter Red | Leaf | 30 | 0 |
| | Shoot apex | 82 | 187 |
| | Node | 145 | 89 |
| | Petiole | 90 | 0 |
| Tempo Pink | Leaf | 35 | 0 |
| | Shoot apex | 196 | 333 |
| | Node | 418 | 184 |
| | Petiole | 259 | 5 |

Transformation of begonia (Begonia Semperflorens) was conducted using breed types Ambassador White and Ambassador Scarlet (Sakata Seed Corporation). Leaf disc and petiole were cut from in vitro seedling of begonia. The samples were infected with Agrobacterium (Agrobacterium tumefaciens Ag10 strain) into which pSPB2314 was introduced, and cultured on a coculture medium (MS medium to which 0.5mg/L of 1AA, 0.1mg/L of TDZ, 0.5% PVP, 2mg/L of AgNO3, 200µM Acetosyringone, and 0.3% gellangum were added) under darkness for 3 days. After that, the samples were sequentially cultured on a preselection medium (MS medium to which 1 mg/L of BAP,1 mg/L of Zeatin, 0.1mg/L of IAA, 500mg/L of Timentin, 50µM Acetosyringone, and 0.25% gellangum were added) for 3 to 5 days, on selective medium 1 (MS medium to which 2mg/L of TDZ, 0.1mg/L of NAA, 100mg/L of Kanamycin, 500mg/L of Timentin, and 0.4% agar were added) for 2 weeks, on selective medium 2 (MS medium to which 0.2mg/L of BAP, 0.1mg/L of NAA, 100mg/L of Kanamycin, 500mg/L of Timentin, and 0.4% agar were added) for 2 weeks, and on selective medium 3 (MS medium to which 100mg/L of Kanamycin, 500mg/L of Timentin, and 0.4% agar were added) for 3 weeks. When a shoot was formed during the culture and grew to have the diameter of 5 mm, its circumjacent tissue was scraped and transferred to selective medium 4 (MS medium to which 150mg/L of Kanamycin, 500mg/L of Timentin, and 0.4% agar were added), and further cultured for 2 to 3 weeks. After that, the tissue was transferred to a rooting medium (MS medium to which 100mg/L of Kanamycin, 500mg/L of Timentin, and 0.4% agar were added) to obtain a rooting shoot (Table 8).

**Table 8**

| Number of shoots of begonia | | |
|---|---|---|
| Breed type | Number of infected explants | Number of shoots obtained |
| Ambassador White | 940 | 13 |
| Ambassador Scarlet | 805 | 2 |

### (PHR1 gene introduction into nicotiana, verbena and cupflower)

Transformation of nicotiana (Nicotiana tabacum), verbena (Verbena hybrida) and cupflower (Nierembergia sp.) was respectively conducted based on publicly-known methods (Science 227, 1229, 1985; Plant Cell Rep. 21, 459, 2003; and Plant Biotech. 23, 19, 2006). Gene introduction of the obtained plants was confirmed by extracting DNA from leaves of each plant body and using PCR in which PHR1 gene was employed as the template. 11 individuals of nicotiana, 16 individuals of verbena and 1 individual of cupflower were obtained as PHR1 transformed plants.

### (PHR1 gene introduction into rose)

Calluses of rose (Rosa hybrida) induced from leaves of aseptic seedling were immersed in a bacteria solution of transformed Agrobacterium having pSPB2314 for 5 minutes. After wiping out excess bacteria solution using sterile filter paper, the calluses were transplanted to a subculture medium and co-cultured in the dark for 2 days. After that, the calluses were washed with MS liquid medium to which 400 mg/l of carbenicillin was added, and transplanted to a medium for selection/bacteria elimination in which 50 mg/l of Kanamycin and 200 mg/l of carbenicillin were added to the subculture medium. Parts which were normally proliferated without growth inhibition on the selection medium were repeatedly transplanted and cultured to select Kanamycin-resistant calluses. By culturing transformed calluses which showed Kanamycin resistance on a redifferentiation medium to which 50 mg/l of Kanamycin and 200 mg/l of carbenicillin were added, Kanamycin-resistant shoot rudiments were obtained. Therefore, there is a high probability that PHR1 transformed plant can also be obtained in the case of rose.

### (Phosphate absorption test for transformed nicotiana and transformed verbena)

Leaves were sampled from aseptic seedlings of PHR1 transformed nicotiana grown on MS medium, and the expression level of PHR1 was confirmed by means of RT-PCR. The strain which showed high expression level was defined as selected strain. After aseptic seedling of the strain was grown on MS medium, a part of leaves was sampled, and the phosphate concentration in the leaves was measured according to the previously-mentioned method. Further, leaves were sampled from potted individuals of PHR1 transformed verbena as in the case of nicotiana, and the expression level of PHR1 was confirmed by means of RT-PCR. The strain which showed high expression level was defined as selected strain. After leaves of the strain were sampled, the phosphate concentration in the leaves was measured according to the previously-mentioned method.

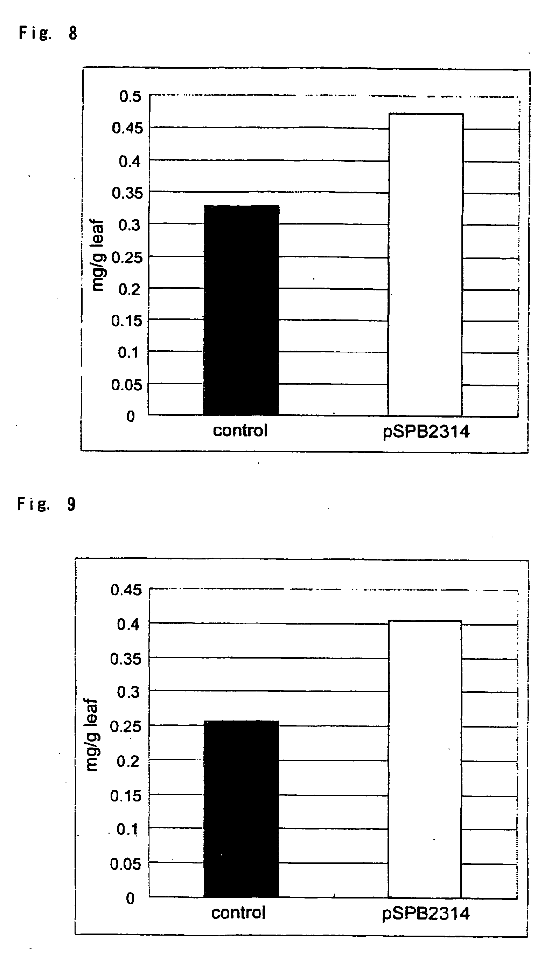

According to the results, both the transformed nicotiana and transformed verbena showed phosphate accumulation about 1.5 times higher than that of the wild type (Figures 8 and 9).

Thus, according to the above-described results, transformed plants into which PHR1 gene was introduced were successfully obtained from 8 plant species, and the phosphate accumulation ability was improved in PHR1-introduced transformed plants of 4 plant species. These results indicate that it is possible to obtain PHR1 transformed plants from various plant species, and that PHR1 gene can contribute to phosphate accumulation regardless of plant species.

### INDUSTRIAL APPLICABILITY

Thus, by utilizing the transformed plant as disclosed herein to which the ability to highly accumulate phosphate is imparted for water purification, collected biomass, which had been a major issue for plants for water purification, can be turned into fertilizer, and can further be effectively reutilized in the form of extracted phosphorus. Moreover, by introducing gene into an existing plant having high phosphate accumulation ability according to this technique, a plant which can accumulate more phosphate can also be prepared. Therefore, with respect to the issue of water purification, the effective technique with little secondary contamination can be provided. Furthermore, by utilizing flowers and ornamental plants for gardening as hosts for gene recombination, advantages from the viewpoint of aesthetic purposes, which other techniques for water purification do not have, can also be provided.

## Claims

1. A water purification method comprising removing excess phosphorus in hydrosphere using a plant body which is transformed by introducing a recombinant expression vector comprising a gene encoding Arabidopsis-derived Myb-like transcription factor PHR1 so that it expresses said gene encoding said transcription factor, wherein the transcription factor is:
(a) a protein consisting of the amino acid sequence set forth in SEQ ID NO: 2; or
(b) a protein, which consists of an amino acid sequence in which one to 20 amino acids are substituted, deleted, inserted or added in the amino acid sequence set forth in SEQ ID NO: 2, and which has the function to activate a phosphate starvation response gene.

2. The method according to claim 1, wherein the gene encoding the Myb-like transcription factor PHR1 is:
(c) a gene comprising an open reading frame of the nucleotide sequence set forth in SEQ ID NO: 1; or
(d) a gene, which hybridizes to a polynucleotide consisting of a nucleotide sequence complementary to the nucleotide sequence set forth in SEQ ID NO: 1 or the nucleotide sequence of the open reading frame thereof under stringent conditions, and which encodes a protein having the function to activate a phosphate starvation response gene.

3. The method according to any one of claims 1 or 2, wherein the plant body is petunia, torenia, verbena, nicotiana, rose, impatiens, begonia or cupflower.

4. Use of a plant body as defined in any one of claims 1 to 3 for removing excess phosphorus in hydrosphere.

## Patentansprüche

1. Verfahren zur Wasseraufreinigung, umfassend das Entfernen von überschüssigem Phosphor in der Hydrosphäre unter Verwendung eines Pflanzenkörpers, der transformiert ist durch das Einbringen eines rekombinanten Expressionsvektors umfassend ein Gen, das einen von Arabidopsis abgeleiteten Myb-like-Transkriptionsfaktor PHR1 codiert, so dass dieser das Gen, das den Transkriptionsfaktor codiert, exprimiert, wobei der Transkriptionsfaktor folgender ist:
(a) ein Protein bestehend aus der in SEQ ID NO:2 dargestellten Aminosäuresequenz; oder
(b) ein Protein, das aus einer Aminosäuresequenz besteht, in der eine bis 20 Aminosäuren in der in SEQ ID NO:2 dargestellten Aminosäuresequenz ersetzt, entfernt, eingefügt oder hinzugefügt sind, und das die Funktion hat, ein Phosphatmangelreaktions-Gen zu aktivieren.

2. Verfahren nach Anspruch 1, wobei das Gen, das den Myb-like-Transkriptionsfaktor PHR1 codiert, folgendes ist:
(c) ein Gen, umfassend einen offenen Leserahmen der in SEQ ID NO:1 dargestellten Nucleotidsequenz; oder
(d) ein Gen, welches an ein Polynucleotid unter stringenten Bedingungen hybridisiert, bestehend aus einer Nucleotidsequenz, die komplementär ist zu der in SEQ ID NO:1 dargestellten Nucleotidsequenz oder zu der Nucleotidsequenz des offenen Leserahmens davon, und das ein Protein codiert, das die Funktion hat, ein Phosphatmangelreaktions-Gen zu aktivieren.

3. Verfahren nach einem der Ansprüche 1 oder 2, wobei der Pflanzenkörper Petunie, Torenie, Verbene, Tabak, Rose, Springkraut, Begonie oder Weißbecher ist.

4. Verwendung eines wie in einem der Ansprüche 1 bis 3 definierten Pflanzenkörpers zum Entfernen von überschüssigem Phosphor in der Hydrosphäre.

## Revendications

1. Procédé de purification d'eau comprenant l'élimination du phosphore en excès dans une hydrosphère en utilisant un organisme végétal qui est transformé en introduisant un vecteur d'expression recombinant contenant un gène codant pour le facteur de transcription PHR1 de type Myb dérivé d'Arabidopsis, de sorte qu'il exprime ledit gène codant pour ledit facteur de transcription, le facteur de transcription étant :
(a) une protéine constituée de la séquence d'acides aminés décrite dans SEQ ID NO: 2 ; ou
(b) une protéine constituée d'une séquence d'acides aminés dans laquelle 1 à 20 acides aminés sont substitués, supprimés, insérés ou ajoutés dans la séquence d'acides aminés décrite dans SEQ ID NO: 2, et dont la fonction consiste à activer un gène de réponse à une privation en phosphate.

2. Procédé selon la revendication 1, dans lequel le gène codant pour le facteur de transcription PHR1 de type Myb est :
(c) un gène comprenant un cadre de lecture ouvert de la séquence de nucléotides décrite dans SEQ ID NO: 1 ; ou
(d) un gène qui s'hybride à un polynucléotide constitué d'une séquence de nucléotides complémentaire à la séquence de nucléotides décrite dans SEQ ID NO: 1 ou à la séquence de nucléotides du cadre de lecture ouvert de celle-ci dans des conditions stringentes, et qui code pour une protéine dont la fonction consiste à activer un gène de réponse à une privation en phosphate.

3. Procédé selon l'une quelconque des revendications 1 ou 2, dans lequel l'organisme végétal est le pétunia, le torénia, la verveine, la nicotiana, la rose, l'impatiens, le bégonia ou le nierembergia.

4. Utilisation d'un organisme végétal tel que défini dans l'une quelconque des revendications 1 à 3 pour éliminer le phosphore en excès dans une hydrosphère.


### SEQUENCE LISTING

<110> Suntory Limited
<120> Plant capable of accumulating inorganic phosphate at high level and use of the plant
<130> G08-0009
<140> PCT/JP2006/322038
   <141> 2006-10-27
<150> IP 2005-313225
   <151> 2005-10-27
<160> 8
<170> PatentIn version 3.3
<210> 1
   <211> 1529
   <212> DNA
   <213> Arabidopsis thaliana
<220>
   <221> CDS
   <222> (99).. (1328)
<400> 1
   ctccgattat ccccaagagg agaatcctca taatcatttt ctccgattcg attcgtcttc      60
   cttggtcctg gattgcttca tgaatttcta ggacaaca atg gag gct cgt cca gtt     116
                                             Met Glu Ala Arg Pro Val
                                             1               5
   cat aga tca ggt tcg aga gac ctc aca cgc act tct tca atc cca tct      164
   His Arg Ser Gly Ser Arg Asp Leu Thr Arg Thr Ser Ser Ile Pro Ser
               10                  15                  20
   aca caa aaa cct tca cca gta gaa gat agt ttc atg aga tca gat aac      212
   Thr Gln Lys Pro Ser Pro Val Glu Asp Ser Phe Met Arg Ser Asp Asn
           25                  30                  35
   aac agt cag tta atg tct aga cca tta gga caa acc tac cat tta ctt      260
   Asn Ser Gln Leu Met Ser Arg Pro Leu Gly Gln Thr Tyr His Leu Leu
       40                  45                  50
   tca tct agt aac ggt gga gct gtt gga cat ata tgt tct tct tca tca      308
   Ser Ser Ser Asn Gly Gly Ala Val Gly His Ile Cys Ser Ser Ser Ser
   55                  60                  65                  70
   tct ggt ttt gca acc aat ctc cat tac tca act atg gta tct cat gag      356
   Ser Gly Phe Ala Thr Asn Leu His Tyr Ser Thr Met Val Ser His Glu
                   75                  80                  85
   aaa caa caa cac tac aca gga agc agc agt aat aat gct gtg cag aca      404
   Lys Gln Gln His Tyr Thr Gly Ser Ser Ser Asn Asn Ala Val Gln Thr
               90                  95                  100
   cca agc aac aac gat agt gct tgg tgt cat gat tca ttg cca gga ggg      452
   Pro Ser Asn Asn Asp Ser Ala Trp Cys His Asp Ser Leu Pro Gly Gly
           105                 110                 115
   ttt ctt gac ttc cat gaa acc aac ccg gcg att caa aac aac tgt cag      500
   Phe Leu Asp Phe His Glu Thr Asn Pro Ala Ile Gln Asn Asn Cys Gln
       120                 125                 130
   att gag gat ggt ggc att gcg gct gct ttt gat gac att caa aaa cga      548
   Ile Glu Asp Gly Gly Ile Ala Ala Ala Phe Asp Asp Ile Gln Lys Arg
   135                 140                 145                 150
   agt gat tgg cat gaa tgg gct gac cat ttg atc act gat gat gat cct      596
   Ser Asp Trp His Glu Trp Ala Asp His Leu Ile Thr Asp Asp Asp Pro
                   155                 160                 165
   ttg atg tct act aac tgg aat gat ctc ttg ctt gaa aca aat tcc aat      644
   Leu Met Ser Thr Asn Trp Asn Asp Leu Leu Leu Glu Thr Asn Ser Asn
               170                 175                 180
   tca gat tca aag gac cag aag aca ctg caa att ccg caa cct cag att      692
   Ser Asp Ser Lys Asp Gln Lys Thr Leu Gln Ile Pro Gln Pro Gln Ile
           185                 190                 195
   gtt cag cag caa cct tct ccg tct gtg gaa ttg cga cct gtt agc aca      740
   Val Gln Gln Gln Pro Ser Pro Ser Val Glu Leu Arg Pro Val Ser Thr
       200                 205                 210
   aca tct tca aac agc aat aac gga acg ggc aag gca cga atg cgt tgg      788
   Thr Ser Ser Asn Ser Asn Asn Gly Thr Gly Lys Ala Arg Met Arg Trp
   215                 220                 225                 230
   acg cca gag ctt cac gag gct ttt gtt gag gct gtc aac agt ctt ggc      836
   Thr Pro Glu Leu His Glu Ala Phe Val Glu Ala Val Asn Ser Leu Gly
                   235                     240                     245
   ggt agt gaa aga gct act cct aaa ggg gta ctg aag att atg aaa gtt        884
   Gly Ser Glu Arg Ala Thr Pro Lys Gly Val Leu Lys Ile Met Lys Val
               250                     255                     260
   gaa ggc ttg act ata tat cat gtt aaa agc cat tta cag aaa tat agg        932
   Glu Gly Leu Thr Ile Tyr His Val Lys Ser His Leu Gln Lys Tyr Arg
           265                     270                     275
   aca gct aga tat cgg cca gaa cca tca gaa act ggt tcg cca gaa agg        980
   Thr Ala Arg Tyr Arg Pro Glu Pro Ser Glu Thr Gly Ser Pro Glu Arg
       280                     285                     290
   aag ttg aca ccg ctt gaa cat ata aca tct ctt gat ttg aaa ggt ggg       1028
   Lys Leu Thr Pro Leu Glu His Ile Thr Ser Leu Asp Leu Lys Gly Gly
   295                     300                     305                     310
   ata ggt att aca gag gct cta cga ctt cag atg gaa gta cag aag caa       1076
   Ile Gly Ile Thr Glu Ala Leu Arg Leu Gln Met Glu Val Gln Lys Gln
                   315                     320                     325
   ctc cat gag cag ctc gag att caa aga aac ctg caa ctc cga ata gaa       1124
   Leu His Glu Gln Leu Glu Ile Gln Arg Asn Leu Gln Leu Arg Ile Glu
               330                     335                     340
   gaa caa ggc aag tac ctg caa atg atg ttc gag aag caa aac tct ggt       1172
   Glu Gln Gly Lys Tyr Leu Gln Met Met Phe Glu Lys Gln Asn Ser Gly
           345                     350                     355
   ctt acc aaa ggg aca gcc tca aca tca gat tcc gca gcc aaa tct gaa       1220
   Leu Thr Lys Gly Thr Ala Ser Thr Ser Asp Ser Ala Ala Lys Ser Glu
       360                     365                     370
   caa gaa gac aag aag act gct gat tcg aag gag gtt cca gaa gaa gaa       1268
   Gln Glu Asp Lys Lys Thr Ala Asp Ser Lys Glu Val Pro Glu Glu Glu
   375                     380                     385                     390
   acc agg aaa tgt gag gaa cta gaa tct cca cag cca aag cgt ccc aaa       1316
   Thr Arg Lys Cys Glu Glu Leu Glu Ser Pro Gln Pro Lys Arg Pro Lys
                   395                     400                     405
   atc gat aat tga aagtattggt cttttgctgg ataatctcgg agtttcagag             1368
   Ile Asp Asn
   ttaacagtga tagagagaac gagctcttat cttgaggttc ttcaggactt ctctctttac     1428
   tgtttaacta tttttctgat ggaagactct caactcagct gtgtagacaa acaacagatc 1488
   cccaaggaag aaacaaaaca aaaataaaaa gttagtttgg g 1529
<210> 2
   <211> 409
   <212> PRT
   <213> Arabidopsis thaliana
<400> 2
   Met Glu Ala Arg Pro Val His Arg Ser Gly Ser Arg Asp Leu Thr Arg
   1               5                   10                  15
   Thr Ser Ser Ile Pro Ser Thr Gln Lys Pro Ser Pro Val Glu Asp Ser
               20                  25                  30
   Phe Met Arg Ser Asp Asn Asn Ser Gln Leu Met Ser Arg Pro Leu Gly
           35                  40                  45
   Gln Thr Tyr His Leu Leu Ser Ser Ser Asn Gly Gly Ala Val Gly His
       50                  55                  60
   Ile Cys Ser Ser Ser Ser Ser Gly Phe Ala Thr Asn Leu His Tyr Ser
   65                  70                  75                  80
   Thr Met Val Ser His Glu Lys Gln Gln His Tyr Thr Gly Ser Ser Ser
                   85                  90                  95
   Asn Asn Ala Val Gln Thr Pro Ser Asn Asn Asp Ser Ala Trp Cys His
               100                 105                 110
   Asp Ser Leu Pro Gly Gly Phe Leu Asp Phe His Glu Thr Asn Pro Ala
           115                 120                 125
   Ile Gln Asn Asn Cys Gln Ile Glu Asp Gly Gly Ile Ala Ala Ala Phe
       130                 135                 140
   Asp Asp Ile Gln Lys Arg Ser Asp Trp His Glu Trp Ala Asp His Leu
   145 150 155 160
   Ile Thr Asp Asp Asp Pro Leu Met Ser Thr Asn Trp Asn Asp Leu Leu
   165 170 175
   Leu Glu Thr Asn Ser Asn Ser Asp Ser Lys Asp Gln Lys Thr Leu Gln
   180 185 190
   Ile Pro Gln Pro Gln Ile Val Gln Gln Gln Pro Ser Pro Ser Val Glu
   195 200 205
   Leu Arg Pro Val Ser Thr Thr Ser Ser Asn Ser Asn Asn Gly Thr Gly
   210 215 220
   Lys Ala Arg Met Arg Trp Thr Pro Glu Leu His Glu Ala Phe Val Glu
   225 230 235 240
   Ala Val Asn Ser Leu Gly Gly Ser Glu Arg Ala Thr Pro Lys Gly Val
   245 250 255
   Leu Lys Ile Met Lys Val Glu Gly Leu Thr Ile Tyr His Val Lys Ser
   260 265 270
   His Leu Gln Lys Tyr Arg Thr Ala Arg Tyr Arg Pro Glu Pro Ser Glu
   275 280 285
   Thr Gly Ser Pro Glu Arg Lys Leu Thr Pro Leu Glu His Ile Thr Ser
   290 295 300
   Leu Asp Leu Lys Gly Gly Ile Gly Ile Thr Glu Ala Leu Arg Leu Gln
   305 310 315 320
   Met Glu Val Gln Lys Gln Leu His Glu Gln Leu Glu Ile Gln Arg Asn
   325 330 335
   Leu Gln Leu Arg Ile Glu Glu Gln Gly Lys Tyr Leu Gln Met Met Phe
   340 345 350
   Glu Lys Gln Asn Ser Gly Leu Thr Lys Gly Thr Ala Ser Thr Ser Asp
   355 360 365
   Ser Ala Ala Lys Ser Glu Gln Glu Asp Lys Lys Thr Ala Asp Ser Lys
   370 375 380
   Glu Val Pro Glu Glu Glu Thr Arg Lys Cys Glu Glu Leu Glu Ser Pro
   385 390 395 400
   Gln Pro Lys Arg Pro Lys Ile Asp Asn
   405
<210> 3
   <211> 21
   <212> DNA
   <213> Artificial
<220>
   <223> primer PHRf
<400> 3
   atggaggctc gtccagttca t 21
<210> 4
   <211> 22
   <212> DNA
   <213> Artificial
<220>
   <223> primer PHRr
<400> 4
   tcaattatcg attttgigac gc 22
<210> 5
   <211> 26
   <212> DNA
   <213> Artificial
<220>
   <223> primer BamPHRf
<400> 5
   agctggatcc alggaggctc gtccag 26
<210> 6
   <211> 26
   <212> DNA
   <213> Artificial
<220>
   <223> primer SpePHRr
<400> 6
   cagtactagt attatcgatt ttggga 26
<210> 7
   <211> 26
   <212> DNA
   <213> Artificial
<220>
   <223> primer SpeVP16f
<400> 7
   agctactagt gcccccccga ccgatg 26
<210> 8
   <211> 26
   <212> DNA
   <213> Artificial
<220>
   <223> primer KpnVP16r
<400> 8
   cagtggtacc ttacccaccg tactcg 26